Europäisches Patentamt

**European Patent Office**

**Office européen des brevets**

(11) Numéro de publication: **0 273 817**
**A1**

(12) # DEMANDE DE BREVET EUROPEEN

(21) Numéro de dépôt: **87402780.8**

(51) Int. Cl.⁴: **C 07 C 6/04**
**B 01 J 23/92**

(22) Date de dépôt: **08.12.87**

(30) Priorité: **17.12.86 FR 8617786**

(43) Date de publication de la demande:
**06.07.88 Bulletin 88/27**

(84) Etats contractants désignés: **DE GB IT NL**

(71) Demandeur: **INSTITUT FRANCAIS DU PETROLE**
**4, Avenue de Bois-Préau**
**F-92502 Ruell-Malmalson (FR)**

(72) Inventeur: **De Bonneville, Jean**
**290, Avenue Napoléon Bonaparte**
**F-92500 Rueil-Malmaison (FR)**

**Chauvin, Yves**
**64, avenue du Général Leclerc**
**F-78230 Le Pecq (FR)**

**Gaillard, Jean**
**rue du Commandant Charcot**
**F-69000 Lyon (FR)**

**Gracco, Francis**
**30, rue Martial Marigné**
**F-78360 Montesson (FR)**

**Ham, Pierre Domaine Saint François d'Assise**
**Les Choucas No. 2**
**F-78170 La Celle Saint Cloud (FR)**

(54) Procédé de métathèse d'oléfines.

(57) L'invention concerne un procédé de métathèse d'oléfines notamment d'éthylène et de butène-2 en vue d'obtenir du propylène.

Le procédé est caractérisé en ce qu'il est effectué en présence d'un catalyseur au rhénium dans une zone de réaction à lit mobile, à une température relativement basse entre 0 et 100°C, le catalyseur étant ensuite réoxydé en deux étapes à des températures différentes.

EP 0 273 817 A1

## Description

La présente invention concerne la réaction de métathèse d'oléfines et plus particulièrement la réaction (1) suivante :

éthylène + butène-2 ⇌ 2 propylène   (1)

On peut utiliser au titre de charge des composés riches en éthylène et butène-2.

Dans l'art antérieur, les réactions de métathèse sont généralement effectuées en présence d'un catalyseur à base d'un oxyde de molybdène ou d'un oxyde de tungstène. Ces réactions sont conduites alors à haute température de l'ordre de 250 à 350°C, voire davantage et de ce fait se déroulent essentiellement en phase vapeur. Il est généralement préférable ensuite de procéder à une réoxydation des composés du molybdène ou du tungstène. Cette opération s'effectue dans le réacteur lui-même à une température souvent supérieure à 500°C, par exemple de l'ordre de 550-800°C. Ce système de réoxydation présente de ce fait des inconvénients notamment parce que d'une part, il oblige à arrêter la réaction donc à immobiliser le réacteur (ce qui nécessite en pratique d'utiliser un train d'au moins deux réacteurs de façon à ce que en permanence, il y ait toujours au moins réacteur en fonctionnement) et d'autre part de procéder à une élévation de température du réacteur au moment de la réoxydation du composé métallique (puisque souvent la température réactionnelle est de l'ordre de 300-350°C et celle de la réoxydation est de l'ordre de 500-700°C) et inversement à une diminution de la température pour revenir en marche réactionnelle. Ces montées et descentes de température sont toujours très longues et retardent d'autant la remise en route du réacteur.

Des inconvénients accrus existent lorsqu'on effectue la métathèse à basse température, avec un catalyseur au rhénium

L'objet, de l'invention permet la suppression de tous ces inconvénients.

On utilise un catalyseur particulier à base d'un oxyde de rhénium déposé sur de l'alumine ou un support ou composé dérivé de l'alumine, par exemple silice-alumine ou oxyde de bore-alumine. Le choix de ce catalyseur permet d'opérer le réacteur à une température de l'ordre de 0 à 100°C, de préférence 25 à 75°C seulement ; de ce fait, la réaction se déroule de préférence en phase liquide et permet alors d'utiliser au moins en réacteur à lit mobile de catalyseur, la charge circulant soit de haut en bas soit de bas en haut. En cas de travail en phase liquide, on opère avec une VVH comprise de préférence entre 0,1 et 10 sous une pression comprise de préférence, entre 20 et 80 bars. Dans un tel système, le catalyseur n'est plus réoxydé dans le réacteur lui-même mais dans une zone de réoxydation distincte de la zone de régénération. Ainsi le système selon l'invention permet de ne plus avoir un réacteur en attente et le problème des températures différentes dans le réacteur et dans la zone de réoxydation du catalyseur ne se pose plus puisque les xones de réaction et de réoxydation sont maintenant indépendantes les unes des autres.

Par réacteur à lit mobile de catalyseur, on entend un réacteur dans lequel le soutirage de catalyseur s'effectue sans interruption de la réaction, ce soutirage étant soit permanent soit périodique, à périodicité courte, inférieure à 12 heures, ce soutirage ne concernant qu'une fraction du catalyseur de manière qu'il subsiste toujours un lit de catalyseur dans le réacteur.

D'une façon préférée, de catalyseur soutiré en continu ou périodiquement de la zone de réaction est envoyé (après avoir été séparé de l'effluent réactionnel) par exemple par un moyen élévateur dans un ballon accumulateur d'où il est envoyé, généralement par gravité, dans au moins deux zones superposées de réoxydation du composé de rhénium. Dans la première zone de réoxydation, dite zone supérieure, le composé de rhénium peut être traité entre 350 et 750°C par un gaz renfermant 1 à 5% d'oxygène moléculaire pendant au moins 10 minutes, par exemple 1-12 heures, et dans la deuxième zone de réoxydation dite zone inférieure, le composé d rhénium peut être traité, entre 400 et 800°C, à une température supérieure à la température de la zone supérieure (de l'ordre de 50 à 200°C supérieure à la température de la zone supérieure) par un gaz renfermant 5 à 40% (de préférence 15 à 25%) d'oxygène moléculaire (de l'air par exemple), pendant au moins 10 minutes par exemple 1-12 heures, le catalyseur réoxydé étant renvoyé ensuite (par exemple par un moyen élévateur) en tête de la zone de réaction, après avoir été purgé et refroidi par exemple au moyen d'un gaz inerte (azote par exemple).

Le temps mis par le catalyseur pour circuler de haut en bas dans la zone de réaction dépend de la température réactionnelle. A titre d'exemple, il est de 8 jours environ à 30°C et de 2 jours environs à 70°C. A ce stade le catalyseur à généralement perdu la moitié environ de son activité initiale. Cette désactivation n'est pas nécessairement liée aux impuretés présentes dans la charge oléfinique mais peut être due à une réduction, par les réactifs, de l'espèce active qui existe à un degré d'oxydation élevé (Re VII, Re VI).

Le catalyseur utilisé dans l'invention est obtenu par imprégnation d'un support par une solution généralement aqueuse d'un composé de rhénium, par exemple l'acide perrhénique, ou l'un de ses sels, l'anhydride perrhénique etc..., de façon à obtenir une concentration comprise de préférence entre 1 et 20% de rhénium (exprimé en métal) en poids par rapport au support. Le catalyseur peut être activé par un traitement progressif entre 500 et 800°C par un courant de gaz sec contenant de l'oxygène moléculaire (air par exemple) puis est refroidi sous un courant de gaz inerte (azote par exemple).

## Exemple

100 volumes d'un catalyseur, constitué d'alumine sous forme de billes imprégnées par du perrhenate d'ammonium (teneur en rhénium métal : 3,85 % en poids), activé à l'air sec à 120°C et ensuite à 500°C, sont introduits sous atmosphère d'azote dans un réacteur cylindrique vertical d'une capacité légèrement supérieure à 100 volumes.

Dans ce réacteur, maintenu entre 35 et 45°C et sous une pression de 3,5 M pa, on fait passer de bas en haut un mélange liquide équimolaire (33 / 66 en poids) d'éthylène et de butène-2 au débit de 300 volumes/ heure. Quand la conversion de l'éthylène et du butène se stabilise à environ 50% (le mélange de sortie contenant 50% en poids de propylène), on soutire toutes les 20 minutes, en bas du réacteur, 0,46 volume de catalyseur qui est envoyé dans un pot de détente dans lequel la pression est ramenée à la pression atmosphérique. Le gaz issu du pot de détente est recomprimé a 3,5 M Pa et liquéfié. Il servira à véhiculer le catalyseur réoxydé vers le réacteur. Les dernières traces d'oléfines sont alors éliminées par balayage d'azote.

Le catalyseur est ensuite introduit, par gravité, et toutes les 20 minutes dans le cylindre supérieur d'un système de réoxydation constitué de 2 cylindres verticaux superposés maintenus à une pression voisine de la pression atmosphérique. Le cylindre supérieur d'une capacité de 10 volumes est parcouru de bas en haut par un courant gazeux sec constitué d'un mélange d'air et d'azote porté à 400°C et contenant environ 2% volume d'oxygène. Après 20 minutes le catalyseur est transféré dans le cylindre inférieur d'une capacité de 5 volumes et parcouru de haut en bas par un courant d'air sec (teneur en eau inférieure à 200 ppm) porté à 550°C.

Après 20 minutes le catalyseur s'écoule au travers d'un système de 12 tubes refroidis par des ailettes de façon à ramener sa température à moins de 40°C et tombe dans un pot parcouru par un courant d'azote sec à un débit tel que la teneur en oxygène du gaz de sortie soit inférieure à 0,2% en volume.

De là, au travers d'une vanne, le catalyseur tombe dans un dernier pot dans lequel est introduit le mélange d'éthylène, de propylène et de butène-2 issu du pot de détente, et qui a été reliquéfié à 3,5 M Pa.

Le gaz liquéfié entraîne le catalyseur ainsi regénéré en haut du réacteur.

## Revendications

1/ Procédé de métathèse d'oléfines en présence d'un catalyseur à base d'oxyde de rhénium déposé sur un support renfermant au moins de l'alumine ou un composé d'alumine, la réaction étant effectuée entre 0 et 100°C dans au moins une zone de réaction de type à lit mobile et étant suivie de la réoxydation et de la réutilisation du catalyseur, caractérisé en ce que catalyseur soutiré de la zone de réaction est réoxyde hors de la zone de réaction par mise en contact avec successivement un gaz renfermant 1 à 5% d'oxygène moléculaire, à une première température entre 350 et 750°C, puis avec un gaz renfermant 5 à 40% d'oxygène moléculaire à une seconde température entre 400 et 800°C, ladite seconde température étant d'au moins 50°C supérieure à ladite première température, et en ce que le catalyseur réoxydé est ramené à la température de la métathèse et renvoyé à la zone de réaction pour être utilisé à nouveau pour la métathèse.

2/ Procédé selon la revendication 1 caractérisé en outre en ce que le catalyseur soutiré de la zone de réaction est envoyé dans une zone d'accumulation, de laquelle il descend par gravité dans au moins deux zones superposées de réoxydation du catalyseur, le catalyseur étant traité dans la zone supérieure entre 350 et 750 °C au moyen d'un gaz renfermant 1 à 5% d'oxygène moléculaire pendant au moins 10 minutes, le catalyseur étant traité ensuite dans la zone inférieure à une température comprise entre 400 et 800°C et supérieure à la température de la zone supérieure au moyen d'un gaz renfermant 5 à 40% d'oxygène moléculaire pendant au moins 10 minutes, le catalyseur ainsi traité étant refroidi au moyen d'un gaz inerte et renvoyé en tête de la zone de réaction.

3/ Procédé selon la rev. 1 ou 2, dans lequel la seconde mise en contact du catalyseur soutiré de la zone de réaction avec un gaz renfermant de l'oxygène à 400-800°C s'effectue avec un gaz renfermant 15 à 25% d'oxygène moléculaire.

4/ Procédé selon l'une des rev. 1 à 3, dans lequel la réaction de métathèse est mise en oeuvre en phase liquide à une VVH entre 0,1 et 10 sous une pression entre 20 et 80 bars.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 027 534  (THE BRITISH PETROLEUM CO.)<br><br>----- | | C 07 C   6/04<br>B 01 J  23/92 |

**DOMAINES TECHNIQUES RECHERCHES (Int. Cl.4)**

C 07 C   6/00
B 01 J  23/00

Le présent rapport a été établi pour toutes les revendications

| Lieu de la recherche | Date d'achèvement de la recherche | Examinateur |
|---|---|---|
| LA HAYE | 02-03-1988 | VAN GEYT J.J.A. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
..............................................................................................
& : membre de la même famille, document correspondant

EPO FORM 1503 03.82 (P0402)